# EUROPEAN PATENT SPECIFICATION

(11) **EP 2 759 532 B1**
(45) Date of publication and mention of the grant of the patent: **31.08.2016**
(21) Application number: 13781207.9
(22) Date of filing: 27.04.2013
(51) Int. Cl.: C07C 323/22, C07C 323/52, C07C 323/58, C07C 319/18, C07C 319/20, C07C 45/52, C07C 47/22, C07C 319/28

(54) **TREATING METHOD AND SYSTEM FOR CRUDE METHYLTHIOPROPANAL SYNTHESIZED OF METHYL MERCAPTAN AND ACRALDEHYDE**
VERFAHREN UND SYSTEM FÜR AUS METHYLMERCAPTAN UND ACRALDEHYD SYNTHETISIERTES ROH-METHYLTHIOPROPANAL
PROCÉDÉ ET SYSTÈME DE TRAITEMENT DE MÉTHYLTHIOPROPANAL BRUT SYNTHÉTISÉ À PARTIR DE MÉTHYLMERCAPTAN ET D'ACRALDÉHYDE

(30) Priority: 28.04.2012 CN 201210131610
(43) Date of publication of application: 30.07.2014
(73) Proprietor: Ningxia Unisplendour Tianhua Methionine Co. Ltd., Zhongwei City Ningxia 755000 (CN)
(72) Inventor: LUO, Yucheng, Chongqing 408200 (CN); JU, Yuanbo, Chongqing 408200 (CN); WANG, Xiaoyong, Chongqing 408200 (CN); MA, Xiuqi, Chongqing 408200 (CN); LIU, Banglin, Chongqing 408200 (CN); WANG, Rongxue, Chongqing 408200 (CN)
(74) Representative: ABG Patentes, S.L.
(86) International application number: PCT/CN2013/074875
(87) International publication number: WO 2013/159744

(56) References cited:
- CN-A- 102 282 119
- CN-A- 102 633 698
- FR-A- 1 535 386
- FR-A1- 2 938 535
- FR-A5- 2 163 119

## Description

### FIELD OF THE INVENTION

The invention relates to treatment of crude methylthiopropanal synthesized through reaction of methyl mercaptan and acraldehyde, and particularly relates to a treating method and system of byproduct in crude methylthiopropanal.

### BACKGROUND OF THE INVENTION

Methylthiopropanal is an important intermediate for the preparation of methionine, and is mainly prepared through Michael addition reaction of methyl mercaptan and acraldehyde under the catalysis of an organic base. By this method, methylthiopropanal can be prepared under relatively mild conditions, for example, a temperature of 30-40 °C and normal pressure, with a yield more than 90%. However, a plurality of byproducts of acraldehyde polymer and methylthiopropanal polymer, including dimers, trimers and the like, would generate in the reaction process, and meanwhile a small amount of dimethyl sulfide, hydrogen sulfide, water, methanol and acetic acid that do not participate in the reaction and methyl mercaptan and acraldehyde that do not react completely are present. Some organic substances among them are light components having a boiling point lower than that of methylthiopropanal, such as dimethyl sulfide, hydrogen sulfide, methyl mercaptan and acetic acid; and the other organic substances are heavy components having a boiling point higher than that of methylthiopropanal, such as acraldehyde polymer and methylthiopropanal polymer, including dimers, trimers and the like. Due to the relatively high boiling point of methylthiopropanal, reduced-pressure rectification is adopted generally. In order to prevent polymerization of organic substances having aldehyde groups and ethylenic bonds and decrease in the efficiency of the rectifying column and in the yield of the methylthiopropanal product, in general, the byproducts having a lower boiling point, e.g. the malodorous gases such as hydrogen sulfide, dimethyl sulfide, methyl mercaptan and acraldehyde, are discharged to a light component buffer tank from the top of the low-vacuum rectifying column through a vacuum pump under low vacuum conditions and then subjected to incineration treatment. The substances at the bottom of the column comprise a majority of methylthiopropanal and a small amount of byproducts having a higher boiling point, e.g. acraldehyde polymer and methylthiopropanal polymer, including dimers, trimers and the like, and they are fed to a high-vacuum rectifying column. The methylthiopropanal product is collected from the top of the high-vacuum rectifying column, and the byproducts having a high boiling point from the bottom of the column are fed to a heavy component buffer tank and then subjected to incineration treatment. Most of the light component and the heavy component are sulfur-containing organic substances which have the characteristics of essence and strong adhesion, and they would make a person feel nauseous and ill when the concentration thereof in the air achieves 0.08 ppb. Hence, in the process of refining methylthiopropanal, the byproducts must be controlled strictly and leakage thereof to the air is not allowable. Otherwise, problems of environmental protection will occur, and the living environment of residents will be affected seriously.

In practical industrial production, light component and heavy component are generally conveyed to be incinerated separately. However, as the light component is very viscous like asphalt, when it is conveyed to be incinerated, there is no appropriate pump with good sealability for conveying the viscous substance, and meanwhile blockage of pipeline readily occurs. Once the pipeline is blocked, not only the continuous and stable production in the workshop would be influenced, but also leakage of the heavy component to the environment would occur in the process of overhauling the blocked pipeline, resulting in serious environmental pollution.

FR1535386, FR2163119 and FR2938535 disclose processes for treating crude methylthiopropanal.

### DESCRIPTION OF THE INVENTION

The object of the present invention is to solve the above-described problem and provide a treating method for crude methylthiopropanal synthesized through reaction of methyl mercaptan and acraldehyde, in which the light and heavy components are subjected to incineration treatment after being mixed and emulsified.

According to the first aspect of the present invention, a method for treating crude methylthiopropanal synthesized through reaction of methyl mercaptan and acraldehyde is provided, which comprises:
feeding crude methylthiopropanal into a low-vacuum rectifying column, discharging a light component from the top of the column, feeding the light component into an emulsifying tank, and discharging a heavier component containing methylthiopropanal from the bottom of the column;
feeding the heavier component containing methylthiopropanal and discharged from the bottom of the low-vacuum rectifying column into a high-vacuum rectifying column, discharging and collecting the methylthiopropanal product from the top of the column, discharging a heavy component from the bottom of the column and feeding it also into the emulsifying tank; and
emulsifying the light component and the heavy component in the emulsifying tank to form a homogeneous and stable emulsion; and feeding the emulsion into an incinerator through a pipeline.

Preferably, the method of the present invention further comprises feeding a combustion-supporting gas and a fuel gas into the incinerator to assist incineration of the emulsion.

The emulsifying tank is provided externally with a layer of thermal-insulation jacket, and provided internally with a stirrer and a transmission for controlling the stirrer.

The rotation rate of the stirrer is not less than 150 rpm; the emulsification temperature is not lower than 60 °C; and the emulsification time is not less than 5 min. More preferably, the rotation rate of the stirrer is 200 rpm; the emulsification temperature is 80 °C; and the emulsification time is 10 min.

Preferably, the emulsion particles have a diameter of 100 nm.

Preferably, the emulsion has a viscosity substantially identical to that of the methylthiopropanal product. Preferably, the emulsion is fed by a canned pump into the incinerator through a pipeline.

The vacuum degree of the low-vacuum rectifying column is 8-20 kPa; and the vacuum degree of the high-vacuum rectifying column is 1-5 kPa.

The crude methylthiopropanal generally comprises methylthiopropanal, hydrogen sulfide, dimethyl sulfide, acraldehyde, acetic acid, methanol, water, hydroquinone, morpholine, acraldehyde polymer and methylthiopropanal polymer. The light component generally comprises hydrogen sulfide, dimethyl sulfide, acraldehyde, acetic acid, methanol, morpholine and water. The heavier component generally comprises methylthiopropanal, hydroquinone, acraldehyde polymer and methylthiopropanal polymer. The heavy component generally comprises hydroquinone, a large amount of methylthiopropanal polymer, a small amount of acraldehyde polymer and a small amount of methylthiopropanal.

According to the second aspect of the present invention, a system for treating crude methylthiopropanal synthesized through reaction of methyl mercaptan and acraldehyde is provided, which comprises:
a low-vacuum rectifying column, for receiving crude methylthiopropanl synthesized through reaction of methyl mercaptan and acraldehyde, discharging a heavier component containing methylthiopropanal from the bottom of the column and discharging a light component from the top of the column;
a high-vacuum rectifying column, for receiving the heavier component containing methylthiopropanal and discharged from the bottom of the low-vacuum rectifying column, discharging the methylthiopropanal product from the top of the column, and discharging a heavy component from the bottom of the column;
an emulsifying tank, for receiving the light component discharged from the top of the low-vacuum rectifying column and the heavy component discharged from the bottom of the high-vacuum rectifying column, wherein the light component and the heavy component are emulsified in the emulsifying tank to form a homogeneous and stable emulsion; and
an incinerator, for receiving the emulsion from the emulsifying tank through a pipeline and incinerating the emulsion.

In the system for treating crude methylthiopropanal of the present invention, it is preferred that the emulsifying tank is provided externally with a layer of thermal-insulation jacket and provided internally with a stirrer and a transmission for controlling the stirrer.

The system for treating crude methylthiopropanal of the present invention may further comprise a canned pump for feeding the emulsion from the emulsifying tank to the incinerator through a pipeline.

By emulsifying the light component and heavy component into an emulsion, the method for treating crude methylthiopropanal synthesized through reaction of methyl mercaptan and acraldehyde of the present invention solves the problem of pipeline blockage caused when the light component and heavy component are conveyed separately, avoids environmental pollution and improves the environments of production workshops and local residents.

### DESCRIPTIONS OF THE FIGURES

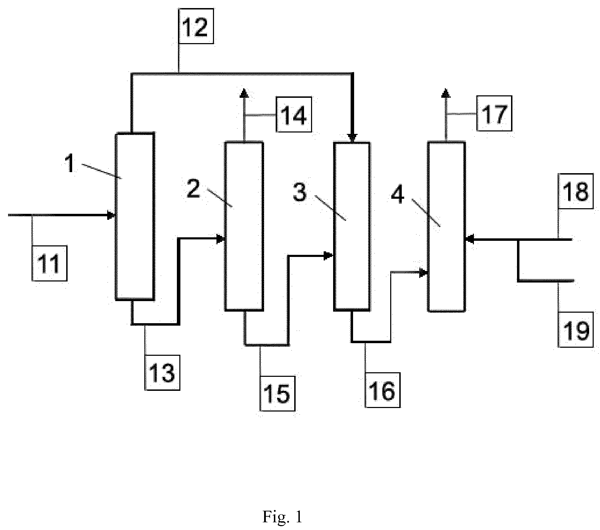

Fig. 1 shows schematic diagram of the process flow for treating crude methylthiopropanal synthesized through reaction of methyl mercaptan and acraldehyde.

### DESCRIPTIONS OF THE PREFERRED EMBODIMENTS

The contents of the present invention are described in detail in combination with the figure and examples.

Referring to the schematic diagram of the process flow for treating crude methylthiopropanal synthesized through reaction of methyl mercaptan and acraldehyde shown in Fig. 1, crude methylthiopropanal 11 mainly comprises methylthiopropanal, hydrogen sulfide, dimethyl sulfide, acraldehyde, acetic acid, methanol, water, hydroquinone, morpholine, acraldehyde polymer and methylthiopropanal polymer, etc. The crude methylthiopropanal 11 is fed to a low-vacuum rectifying column 1 continuously through a pump; a light component 12 having a boiling point lower than that of methylthiopropanl and comprising hydrogen sulfide, dimethyl sulfide, acraldehyde, acetic acid, methanol, morpholine and water is discharged from the top of the low-vacuum rectifying column 1 under a low vacuum condition with vacuum degree of 8-20 kPa, preferably, 15-18 kPa, and directly fed to an emulsifying tank 3. At this time, a heavier component 13 from the bottom of the low-vacuum rectifying column 1, which mainly comprises methylthiopropanal and a heavy component having a boiling point higher than that of methylthiopropanal as well as a small amount of hydroquinone, acraldehyde polymer, methylthiopropanl polymer and the like, is fed to a high-vacuum rectifying column 2 through a pump, and the methylthiopropanal product 14 with a purity greater than 99.7% is discharged from the top of the high-vacuum rectifying column 2 under a high vacuum condition with vacuum degree of 1-5 kPa, preferably 3-4 kPa, and fed to a product tank (not shown in the diagram). The heavy component 15 obtained from the bottom of the high-vacuum rectifying column 2 comprises a large amount of methylthiopropanal polymer, a small amount of acraldehyde polymer, etc. According to the principle of rectifying column and the economical principle for column operation, methylthiopropanal is impossible to be completely distilled out from the top of the column, thus the heavy component from the bottom of the high-vacuum rectifying column 2 further contains a small amount of methylthiopropanal. Since the organic matter in the heavy component 15 from the bottom of the column has the characteristics of essence, and the residue thereof even in a trace amount in the environment would exist almost permanently and pollute the environment. Hence, leakage thereof to the air must be avoided as far as possible. The heavy component 15 is fed to the emulsifying tank 3 through a pump. The emulsifying tank 3 may be provided externally with a layer of thermal-insulation jacket and provided with a stirrer and a continuously variable transmission for controlling the stirrer at its internal part.

When a heavy component 15 is fed to the emulsifying tank 3, mechanical stirring is started. The rotation rate for stirring is adjusted via the continuously variable transmission, and meanwhile the temperature of the emulsifying tank is adjusted. Mechanical stirring is performed till the liquid in the emulsifying tank forms a homogeneous and stable emulsion 16. In this process, the formation of the homogeneous and stable emulsion is crucial. If a homogeneous and stable emulsion cannot be formed, emulsion layering will occur so that the heavy component is disposed in the lower layer and the light weight is disposed in the upper layer. In this way, during the process of conveying the mixture to be incinerated, the heavy component still exhibits a large viscosity and will block the pipeline, thus is unable to be conveyed. Further, in the subsequent process of treating the blocked pipeline, it is inevitable to leak the effluvial gas to the environment and cause serious environmental pollution event. In the method of the present invention, a homogeneous and stable emulsion 16 can be formed by adjusting the rotation rate for stirring, the temperature and the emulsification time; and the emulsion 16 has a viscosity almost equivalent to that of the methylthiopropanal product, and is convenient for conveyance by a canned pump. Table 1 shows the effect of emulsifying the heavy component and light component at various temperatures, rotation rates and emulsification time. The test demonstrates that, the heavy component and light component can be emulsified into an emulsion suitable for conveyance under the conditions of optional form of stirring blade, a rotation rate not less than 150 rpm, an emulsification temperature not lower than 60 °C, and an emulsification time not less than 5 min, wherein the preferable emulsification condition is: a rotation rate of the stirrer of 200 rpm, an emulsification temperature of 80 °C, and an emulsification time of 10 min, and the diameter of the emulsion particles formed under the condition can reach 100 nm.

**Table 1 Emulsifying effect at various temperatures, rotation rates and emulsification time**

| Examples | Rotation rate for stirring rpm | Temper ature °C | Emulsification time min | Emulsion particle diameter nm | Phenomena |
|---|---|---|---|---|---|
| 1 | 60 | 80 | 30 | no | Two phase layering |
| 2 | 150 | 80 | 30 | 400 | A majority of emulsion, and a little heavy component at lower part |
| 3 | 200 | 80 | 10 | 100 | Homogeneous and stable emulsion |
| 4 | 200 | 60 | 30 | 350 | A majority of emulsion, and a little heavy component at lower part |
| 5 | 200 | 80 | 5 | 380 | A majority of emulsion, and a little heavy component at lower part |

The emulsified emulsion 16 is fed to an incinerator 4 by a canned pump through a pipeline. A combustion-supporting gas 18 and a fuel gas 19 are fed to the incinerator 4 to assist incineration of the emulsion 16. The smoke 17 generated in incineration mainly comprise carbon dioxide, air, and trace amount of sulfur dioxide, and can be directly discharged to the air.

It follows that, the light component 12 separated from the top of the low-vacuum rectifying column 1 and the heavy component 15 separated from the bottom of the high-vacuum rectifying column 2 are fed together into the emulsifying tank 3, and by controlling the emulsification conditions, they can be prepared into a homogeneous and stable emulsion having a viscosity equivalent to that of methylthiopropanal and being convenient for conveyance with pump, so as to solve the problem of pipeline blockage caused when the light component and the heavy component are conveyed separately, avoid occurrence of environmental protection event and improve the environments of production workshops and local residents.

Those skilled in the art should understand that the above-mentioned examples are merely used to explain the present invention rather than make any limitation thereto.

## Claims

1. A method for treating crude methylthiopropanal synthesized through reaction of methyl mercaptan and acraldehyde, comprising:
feeding crude methylthiopropanal into a low-vacuum rectifying column, discharging a light component from the top of the column, feeding the light component into an emulsifying tank, and discharging a heavier component containing methylthiopropanal from the bottom of the column;
feeding the heavier component containing methylthiopropanal and discharged from the bottom of the low-vacuum rectifying column into a high-vacuum rectifying column, discharging and collecting the methylthiopropanal product from the top of the column, discharging a heavy component from the bottom of the column and feeding it also into the emulsifying tank;
emulsifying the light component and the heavy component in the emulsifying tank to form a homogeneous and stable emulsion; and
feeding the emulsion into an incinerator through a pipeline;
wherein the emulsifying tank is provided externally with a layer of thermal-insulation jacket, and provided internally with a stirrer and a transmission for controlling the stirrer;
wherein the rotation rate of the stirrer is not less than 150 rpm; the emulsification temperature is not lower than 60 °C; and the emulsification time is not less than 5 min;
wherein the vacuum degree of the low-vacuum rectifying column is 8-20 kPa; and the vacuum degree of the high-vacuum rectifying column is 1-5 kPa.

2. The method according to claim 1, wherein the emulsion particles have a diameter of less than 100 nm.

3. The method according to claim 1, wherein the emulsion has a viscosity identical to that of the methylthiopropanal product.

4. The method according to claim 3, wherein the emulsion is fed by a canned pump into the incinerator through a pipeline.

5. A system for treating crude methylthiopropanal synthesized through reaction of methyl mercaptan and acraldehyde, comprising:
a low-vacuum rectifying column, for receiving crude methylthiopropanl synthesized through reaction of methyl mercaptan and acraldehyde, discharging a heavier component containing methylthiopropanal from the bottom of the column and discharging a light component from the top of the column;
a high-vacuum rectifying column, for receiving the heavier component containing methylthiopropanal and discharged from the bottom of the low-vacuum rectifying column, discharging the methylthiopropanal product from the top of the column, and discharging a heavy component from the bottom of the column;
an emulsifying tank, for receiving the light component discharged from the top of the low-vacuum rectifying column and the heavy component discharged from the bottom of the high-vacuum rectifying column, wherein the light component and the heavy component are emulsified in the emulsifying tank to form a homogeneous and stable emulsion; and
an incinerator, for receiving the emulsion from the emulsifying tank through a pipeline and incinerating the emulsion.

6. The system for treating crude methylthiopropanal according to claim 5, wherein the emulsifying tank is provided externally with a layer of thermal-insulation jacket and provided internally with a stirrer and a transmission for controlling the stirrer.

7. The system for treating crude methylthiopropanal according to claim 5, further comprising a canned pump for feeding the emulsion from the emulsifying tank to the incinerator through a pipeline.

## Patentansprüche

1. Verfahren zur Behandlung von rohem Methylthiopropanal, das durch Reaktion von Methylmercaptan und Acrolein synthetisiert wurde, umfassend:
Zuführen von rohem Methylthiopropanal in eine Niedrigvakuum-Rektifikationssäule, Entlassen einer leichten Komponente aus dem oberen Ende der Säule, zuführen der leichten Komponente in einen Emulsionstank, und Entlassen einer schwereren Komponente, die Methylthiopropanal enthält, aus dem unteren Ende der Säule;
Zuführen der schwereren Komponente, die Methylthiopropanal enthält und aus dem unteren Ende der Niedrigvakuum-Rektifikationssäule entlassen wurde, in eine Hochvakuum-Rektifikationssäule, Entlassen und Auffangen des Methylthiopropanal-Produkts aus dem oberen Ende der Säule, Entlassen einer schweren Komponente vom unteren Ende der Säule und diese ebenfalls in einen Emulsionstank zuführen;
Emulgieren der leichten Komponente und der schwereren Komponente in dem Emulsionstank, um eine homogene und stabile Emulsion zu erhalten; und
Zuführen der Emulsion in einen Verbrennungsofen durch eine Pipeline;
wobei der Emulsionstank auf seiner Außenseite mit einer Schicht einer wärmeisolierenden Ummantelung ausgestattet ist, und intern mit einem Rührer und einem Getriebe zur Kontrolle des Rührers ausgestattet ist;
wobei die Umdrehungszahl des Rührers nicht weniger als 150 Umdrehungen pro Minute beträgt; die Emulgierungstemperatur nicht weniger als 60°C beträgt; und die Emulgationszeit nicht weniger als 5 Minuten beträgt;
wobei der Vakuumbereich der Niedrigvakuum-Rektifikationssäule 8-20 kPa beträgt; und der Vakuumbereich der Hochvakuum-Rektifikationssäule 1-5 kPa beträgt.

2. Verfahren nach Anspruch 1, wobei die Emulsionspartikel einen Durchmesser von weniger als 100 nm aufweisen.

3. Verfahren nach Anspruch 1, wobei die Emulsion eine Viskosität aufweist, die identisch zu der des Methylthiopropanal-Produkts ist.

4. Verfahren nach Anspruch 3, wobei die Emulsion mittels einer Spaltrohrpumpe durch eine Pipeline in den Verbrennungsofen geführt wird.

5. Ein System zur Behandlung von rohem Methylthiopropanal, das durch Reaktion von Methylmercaptan und Acrolein synthetisiert wurde, umfassend:
eine Niedrigvakuum-Rektifikationssäule, zum Empfang von rohem Methylthiopropanal, das durch Reaktion von Methylmercaptan und Acrolein synthetisiert wurde, zum Entlassen einer schwereren Komponente, die Methylthiopropanal enthält, aus dem unteren Ende der Säule, und Entlassen einer leichten Komponente aus dem oberen Ende der Säule;
eine Hochvakuum-Rektifikationssäule, zum Empfang der schwereren Komponente, die Methylthiopropanal enthält und aus dem unteren Ende der Niedrigvakuum-Rektifikationssäule entlassen wurde, Entlassen des Methylthiopropanal-Produkts aus dem oberen Ende der Säule, und Entlassen einer schweren Komponente aus dem unteren Ende der Säule;
ein Emulsionstank, zum Empfang der leichten Komponente, die aus dem oberen Ende der Niedrigvakuum-Rektifikationssäule entlassen wurde, und der schweren Komponente, die aus dem unteren Ende der Hochvakuum-Rektifikationssäule entlassen wurde, wobei die leichte Komponente und die schwere Komponente in einem Emulsionstank emulgiert werden, um eine homogene und stabile Emulsion zu bilden; und
einen Verbrennungsofen, um die Emulsion aus dem Emulsionstank durch eine Pipeline zu empfangen und die Emulsion zu verbrennen.

6. System zur Behandlung von rohem Methylthiopropanal nach Anspruch 5, wobei der Emulsionstank auf seiner Außenseite mit einer Schicht einer wärmeisolierenden Ummantelung ausgestattet ist, und intern mit einem Rührer und einem Getriebe zur Kontrolle des Rührers ausgestattet ist.

7. System zur Behandlung von rohem Methylthiopropanal nach Anspruch 5, weiter umfassend eine Spaltrohrpumpe, um die Emulsion von dem Emulsionstank durch eine Pipeline zu dem Verbrennungsofen zu führen.

## Revendications

1. Procédé de traitement de méthylthiopropanal brut synthétisé par réaction de méthylmercaptan et acraldéhyde, comprenant:
l'alimentation de méthylthiopropanal brut dans une colonne de rectification à vide léger, l'évacuation d'un composant léger depuis la tête de la colonne, l'alimentation du composant léger dans un réservoir d'émulsification, et l'évacuation d'un composant plus lourd contenant du méthylthiopropanal depuis le fond de la colonne ;
l'alimentation du composant plus lourd contenant du méthylthiopropanal et évacué depuis le fond de la colonne de rectification à vide léger dans une colonne de rectification à vide poussé, l'évacuation et la récupération du produit méthylthiopropanal depuis la tête de la colonne, l'évacuation d'un composant lourd depuis le fond de la colonne ainsi que son alimentation dans le réservoir d'émulsification ;
l'émulsification du composant léger et du composant lourd dans le réservoir d'émulsification pour former une émulsion homogène et stable ; et
l'alimentation de l'émulsion dans un incinérateur à travers une conduite ;
dans lequel le réservoir d'émulsification est pourvu à l'extérieur d'une couche d'enveloppe d'isolation thermique et à l'intérieur d'un agitateur et d'une transmission permettant de commander l'agitateur ;
dans lequel la vitesse de rotation de l'agitateur n'est pas inférieure à 150 tour/min ; la température d'émulsification n'est pas inférieure à 60 °C ; et le temps d'émulsification n'est pas inférieur à 5 min ;
dans lequel le degré de vide de la colonne de rectification à vide léger est de 8 à 20 kPa ; et le degré de vide de la colonne de rectification à vide poussé est de 1 à 5 kPa.

2. Procédé selon la revendication 1, dans lequel les particules de l'émulsion présentent un diamètre inférieur à 100 nm.

3. Procédé selon la revendication 1, dans lequel l'émulsion présente une viscosité identique à celle du produit méthylthiopropanal.

4. Procédé selon la revendication 3, dans lequel l'émulsion est alimentée par une pompe étanche dans l'incinérateur à travers une conduite.

5. Système de traitement de méthylthiopropanal brut synthétisé par réaction de méthylmercaptan et acraldéhyde, comprenant :
une colonne de rectification à vide léger, permettant la réception du méthylthiopropanal brut synthétisé par réaction de méthylmercaptan et acraldéhyde, l'évacuation d'un composant plus lourd contenant le méthylthiopropanal depuis le fond de la colonne, et l'évacuation d'un composant léger depuis la tête de la colonne ;
une colonne de rectification à vide poussé, permettant la récupération du composant plus lourd contenant le méthylthiopropanal et évacué depuis le fond de la colonne de rectification à vide léger, l'évacuation du produit méthylthiopropanal depuis la tête de la colonne, et l'évacuation d'un composant lourd depuis le fond de la colonne ;
un réservoir d'émulsification, permettant la récupération du composant léger évacué depuis la tête de la colonne de rectification à vide léger et du composant lourd évacué depuis le fond de la colonne de rectification à vide poussé, dans lequel le composant léger et le composant lourd sont émulsifiés dans le réservoir d'émulsification pour former une émulsion homogène et stable ; et
un incinérateur, permettant la récupération de l'émulsion depuis le réservoir d'émulsification à travers une conduite et l'incinération de l'émulsion.

6. Système de traitement de méthylthiopropanal brut selon la revendication 5, dans lequel le réservoir d'émulsification est pourvu à l'extérieur d'une couche d'enveloppe d'isolation thermique et à l'intérieur d'un agitateur et d'une transmission permettant de commander l'agitateur.

7. Système de traitement de méthylthiopropanal brut selon la revendication 5, comprenant en outre une pompe étanche permettant l'alimentation de l'émulsion depuis lé réservoir d'émulsification dans l'incinérateur à travers une conduite.
